# EUROPEAN PATENT SPECIFICATION

(11) **EP 3 975 866 B1**
(45) Date of publication and mention of the grant of the patent: **10.01.2024**
(21) Application number: 20729020.6
(22) Date of filing: 23.05.2020
(51) Int. Cl.: A61B 8/00, A61B 8/12, A61B 8/08, A61B 1/005, A61B 1/273

(54) **HANDLE ASSEMBLY FOR TRANSESOPHAGEAL ECHOCARDIOGRAPHY**
GRIFFANORDNUNG FÜR DIE TRANSÖSOPHAGEALE ECHOKARDIOGRAPHIE
ENSEMBLE POIGNÉE POUR ÉCHOCARDIOGRAPHIE TRANSOESOPHAGIENNE

(30) Priority: 24.05.2019 US 201962852479 P
(43) Date of publication of application: 06.04.2022
(73) Proprietor: Koninklijke Philips N.V., 5656 AG Eindhoven (NL)
(72) Inventor: PESZYNSKI, Michael, 5656 AE Eindhoven (NL); CASWELL, John, Bench, 5656 AE Eindhoven (NL); D'ANELLO, Joseph, 5656 AE Eindhoven (NL); AGIUS, Daniel, 5656 AE Eindhoven (NL)
(74) Representative: Philips Intellectual Property & Standards
(86) International application number: PCT/EP2020/064344
(87) International publication number: WO 2020/239644

(56) References cited:
- WO-A2-2010/020939
- US-A- 5 402 793
- US-A1- 2004 073 118

## Description

### TECHNICAL FIELD

The present disclosure relates generally to the imaging of the organs of a patient using transesophageal echocardiography (TEE), and the control of the TEE probe via pull cables located in its handle. For example, some embodiments of the present disclosure are suited for controlling the position, orientation, and flexion of a TEE gastroscope probe.

### BACKGROUND

Observing the condition and function of a patient's heart can be a difficult and dangerous procedure. Echocardiography can mitigate risk of injury to the patient by using ultrasonic imaging techniques. In an echocardiogram, a physician uses an ultrasonic probe comprising one or more ultrasonic transducers to obtain images of various angles of the patient's heart. The ultrasonic transducers emit ultrasonic energy in the form of ultrasonic waves to create an image of the heart. Ultrasonic waves are partially reflected by discontinuities arising from tissue structures, red blood cells, and other features of interest. Echoes, or the reflected ultrasonic waves, are received by the ultrasonic transducer and transmitted to a signal processor. The signal processor processes the received ultrasound echoes to produce an image of the heart near the location where the ultrasonic transducer is placed.

One common echocardiography procedure is a transthoracic echocardiogram (TTE), which involves placing an ultrasonic probe on the chest or abdomen of a patient to obtain images of various angles of the patient's heart. Although TTE is a relatively non-invasive procedure, the ultrasonic waves must travel through several layers of tissue and bone to reach the heart, and the echoes must travel back through the same tissue and bone to reach the ultrasonic transducer. These thick layers of tissue and bone can weaken the strength of the echoes and degrade the quality of the image obtained.

Transesophageal echocardiography (TEE) involves obtaining an ultrasonic image of the heart using a TEE probe positioned within the patient's esophagus. TEE probes also use ultrasonic waves to obtain an image of a patient's heart. Transesophageal echocardiography can be advantageous because the heart is located near the esophagus, which can result in higher quality images. To obtain an image using a TEE probe, a physician inserts a gastroscope including an ultrasonic transducer into the patient's esophagus and guides the ultrasonic transducer to an area near the heart. The physician manipulates a distal portion of the gastroscope within the patient's esophagus to guide the ultrasonic transducer into place, and to maintain contact with the esophagus wall to facilitate the transmission and reception of the ultrasonic waves.

It is to be appreciated that such commonly used TEE handles have numerous drawbacks, including cost, durability, reliability, ergonomics, electrical grounding, and otherwise. The handle portion of a conventional TEE probe comprises multiple complex machined parts and assemblies that may require highly precise manufacturing tolerances, expensive materials, and substantial assembly time and labor. For example, the brake assembly may include up to four separate pieces working in conjunction with one another. Articulation of current TEE probes involves a stainless steel pull wire system driven by a nested rack and pinion system made of hardened beryllium copper. These metal components may cause electrical isolation issues. Left / Right and Anterior / Posterior Control wheels are attached to respective pinion shafts and provide user inputs to drive the system. Pinions, racks, housings, and related components are all metallic, as are the outside user handles, and potentially the patient contact handles for the control housing. All internal structures are electrically grounded, while metal handles are electrically isolated from internal structures via a complicated set of static and dynamic overlapping isolating components that provide adequate electrical creepage and clearance throughout the entire range of positions the articulation mechanism can provide. Further isolation is achieved via a distal plastic isolation member attached to the main rack and pinion housing via a dovetail engagement. The distal isolator is prone to failure due to its complicated attachment to the metal housing.

US 5 402 793 A discloses a TEE probe comprising a right-left control knob connected by a shaft to a right-left pulley and a forward-back control knob connected by the shaft to a forward-back pulley. A control cable is wrapped around each pulley, and the ends of the control cables are attached to attachment points at the distal end of a gastroscope. The shaft is carried inside a tube which passes through the center of the forward-back control knob and pulley. A limit stop cam is attached to the end of the tube between the two control knobs, and each control knob has a limit stop pin extending from it.

### SUMMARY

The invention is defined by the independent claim. The dependent claims define advantageous embodiments.

Disclosed is a transesophageal echocardiography (TEE) probe mid-handle assembly. The TEE probe mid-handle assembly includes improved and simplified components, and has particular, but not exclusive, utility for the control of TEE probes for imaging of the human heart.

Embodiments of the present disclosure provide an improved mid-handle assembly for a transesophageal echocardiography (TEE) probe for generating images of an organ that has improved cost and manufacturability properties. The TEE probe mid-handle assembly provides a non-metal design for TEE probe articulation that translates a user's turn of a control knob into linear articular pulls of the catheter. The assembly may include a molded plastic containment frame, which electrically isolates any internal components from metal exterior components, if present. The assembly may also include one or more floating drive shafts for translating knob rotation to pull elements, and one or more non-metallic pull elements in communication with the drive shaft or shafts. The TEE mid-handle assembly will accomplish TEE gastroscope articulation and electrical isolation from grounded structures in a more direct and reliable manner by eliminating the traditional rack and pinions and utilizing a nonmetallic method of translating rotational user control to linearly pull articulation control cables. In addition, a nonmetallic molded control knob, cam, and distal isolator may be employed to nest and capture a pair of articulation control shafts, which has the benefit of naturally isolating the articulation assembly from an external metallic handle, if present.

In an example, the TEE probe can include simplified mechanisms, such as friction belts, timing ladders, pulley cables, timing belts, and tape drives, that replace the rack and pinion system to couple the control knobs to the pull cables that steer the imaging element. In some embodiments, the brake assembly has been simplified and may be a monolithic brake switch (i.e., formed as a single piece). In some embodiments, machined metal components have been replaced with molded plastic components having less precise manufacturing tolerances and improved electrical isolation properties. In some embodiments, multi-part assemblies and enclosures have been replaced by assemblies with a smaller part count, fewer assembly steps, fewer failure points, lighter weight, and improved ease of operation.

In an exemplary embodiment, the transesophageal echocardiography (TEE) probe comprises a containment frame, a first control input located external to the containment frame, a first shaft located internal to the containment frame and coupled to the first control input, a first elongated flexible member comprising a first end and a second end, wherein the first elongated flexible member is wrapped around the first shaft, a first pull cable attachment coupled to the first end of the first elongated flexible member, a second pull cable attachment coupled to the second end of the first elongated flexible member, a first pull cable attached to the first pull cable attachment, and a second pull cable attached to the second pull cable attachment, wherein, when the first control input is actuated, the first shaft is rotated such that the first and second ends of the first elongated flexible member move linearly, causing one of the first or second pull cable to move in a first direction and causing the other of the first or second pull cable to move in an opposite, second direction, and wherein a distal portion of a gastroscope coupled to the first and second pull cables is flexed according to movements of the first and second pull cables.

In some embodiments, the TEE probe further comprises a second control input located external to the containment frame, a second shaft located internal to the containment frame and coupled to the second control input, a second elongated flexible member comprising a first end and a second end, wherein the second elongated flexible member is wrapped around the second shaft, a third pull cable attachment coupled to the first end of the second elongated flexible member, a fourth pull cable attachment coupled to the second end of the second elongated flexible member, a third pull cable attached to the third pull cable attachment, and a fourth pull cable attached to the fourth pull cable attachment, wherein, when the second control input is rotated, the second shaft is rotated such that the first and second ends of the second elongated flexible member move linearly, causing one of the third or fourth pull cable to move in the first direction, and causing the other of the third or fourth pull cable to move in the opposite, second direction, and wherein the distal portion of the gastroscope coupled to the third and fourth pull cables is flexed according to movements of the third and fourth pull cables.

In some embodiments, the TEE probe further comprises a monolithic brake switch that, when rotated into a first position, applies a resistance to rotation of the first and second shafts and, when rotated into a second position, permits the first and second shafts to rotate without the resistance. In some embodiments, at least one of the control inputs, shafts, elongated flexible members, brake switch, or containment frame is nonconductive. In some, at least one of the control inputs, shafts, brake switch, or containment frame is made of molded plastic. In some embodiments, the first and second elongated flexible members are made of elastomeric compounds molded over nylon reinforcement members. In some embodiments, the first control input directs anterior and posterior flexion of the distal portion of the gastroscope, and the second control input directs left/right flexion of the gastroscope. In some embodiments, the first and second elongated flexible members are friction belts. In some embodiments, the first and second shafts comprise gears or pinions, and the first and second elongated flexible members are timing ladder belts interfacing with the gears or pinions. In some embodiments, the first shaft and second shaft each comprise a socket, and wherein the first elongated flexible member is a pulley cable anchored to the first shaft by a swaged ball or screw attachment fitting into the socket in the first shaft, and wherein the second elongated flexible member is a pulley cable anchored to the second shaft by a swaged ball or screw attachment fitting into the socket in the second shaft. In some embodiments, the first and second shafts comprise gears or pinions, and wherein the first and second elongated flexible members are timing belts interfacing with the gears or pinions. In some embodiments, the first and second shafts comprise gears or pinions, and the first and second elongated flexible members are drive tapes interfacing with the gears or pinions.

In some embodiments the pull cable attachments are formed directly into the elongated flexible members. In some embodiments, the pull cable attachments each include a threaded tensioning mechanism. In some embodiments, the TEE probe further comprises an outer handle casing. In some embodiments wherein the first and second shafts are coaxial. In some embodiments, the TEE hand further comprises a floating, nonrotating interior shaft affixed to the containment frame and situated between the first and second shafts to limit cross-coupling of motion between the first and second shafts.

In an exemplary embodiment, a method for transesophageal echocardiography (TEE) comprises controlling flexion of a distal portion of a gastroscope of a TEE probe, based on actuation of a control input of the TEE probe, wherein the TEE probe comprises the gastroscope, a containment frame, a handle assembly coupled to the gastroscope and comprising the control input, a shaft coupled to the control input, an elongated flexible member comprising a first end and a second end, the elongated flexible member wrapped around the shaft, a first pull cable attachment attached to the first end of the elongated flexible member, and a second pull cable attachment attached to the second end of the first elongated flexible member, a first pull cable attached to the first pull cable attachment and the distal portion of the gastroscope; and a second pull cable attached to the second pull cable attachment and the distal portion of the gastroscope, wherein controlling flexion includes: rotating the shaft coupled to the control input, in response to actuation of the control input, linearly moving, in response rotating the shaft, the first pull cable attachment and the second pull cable attachment, linearly moving, in response to corresponding movements of the first pull cable attachment and the second pull cable attachment, the first pull cable and the second pull cable to cause the flexion of the distal portion of the gastroscope, and applying a resistance to rotation of the shaft, in response to rotation of a brake switch of the handle assembly into a first position, and permitting rotation of the shaft without the resistance, in response to the rotation of the brake switch to a second position. In some embodiments, at least one of the brake switch, the control input, the shaft, the elongated flexible member, the containment frame, the first pull cable attachment, or the second pull cable attachment are made of molded plastic.

In an exemplary embodiment, a TEE probe comprises a gastroscope and a handle assembly coupled to the gastroscope and comprising a control input, a shaft coupled to the control input, an elongated flexible member wrapped around the shaft, the elongated flexible member comprising a first end and an opposite, second end, wherein the elongated flexible member comprises at least one of a friction belt, a timing ladder, a pulley cable, a timing belt, or a drive tape, a first pull cable attachment and a second pull cable attachment respectively attached to the first end and the second end of the elongated flexible member, and a monolithic brake switch configured to selectively apply resistance to rotation of the shaft, a first pull cable attached to the first pull cable attachment and a distal portion of the gastroscope, and a second pull cable attached to the second pull cable attachment and the distal portion of the gastroscope for each elongated flexible member, wherein, when the control input is actuated, the shaft rotates such that the first and second pull cable attachments move linearly and such that the first and second pull cables correspondingly move linearly to flex the distal portion of the gastroscope, and wherein the monolithic brake switch, when rotated into a first position, applies the resistance to the rotation of the shaft and, when rotated into a second position, permits the shaft to rotate without the resistance, and wherein at least one of the brake switch, the control input, the shaft, the elongated flexible member, the first pull cable attachment, or the second pull cable attachment are made of molded plastic.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the TEE probe handle assembly, as defined in the claims, is provided in the following written description of various embodiments of the disclosure and illustrated in the accompanying drawings.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
**Figure 1** depicts a diagrammatic view of a transesophageal echocardiography (TEE) ultrasonic imaging system, according to at least one embodiment of the present disclosure.
**Figure 2** is a side elevation view of a TEE probe, according to at least one embodiment of the present disclosure.
**Figure 3** is a diagrammatic view of the handle portion of the TEE probe of Figure 2, according to at least one embodiment of the present disclosure.
**Figure 4** depicts a translucent isometric view of the interior mechanism of a conventional TEE mid-handle assembly.
**Figure 5A** is a side elevation view of a lateral side of a distal portion of a TEE probe, illustrating anterior and posterior flexion of the distal portion, according to at least one embodiment of the present disclosure.
**Figure 5B** is a side elevation view of a front side of the distal portion of the TEE probe of Figure 5A, illustrating left and right flexion of the distal portion, according to at least one embodiment of the present disclosure.
**Figure 5C** is a side elevation view of the front side of the distal portion of the TEE probe of Figure 5A, illustrating counterclockwise rotation, clockwise rotation, advanced and withdrawn positioning of the distal portion, according to at least one embodiment of the present disclosure.
**Figure 5D** is an isometric view of the distal portion of the TEE probe of Figure 5A, illustrating increasing and decreasing multi-plane angles of an ultrasonic transducer, according to at least one embodiment of the present disclosure.
**Figure 6** is an isometric view of a portion of the mid-handle mechanism of an example TEE probe that incorporates friction belts according to at least one embodiment of the present disclosure.
**Figure 7** is an isometric view of a portion of the mid-handle mechanism of an example TEE probe that incorporates friction belts according to at least one embodiment of the present disclosure.
**Figure 8** is an isometric view of a portion of the mid-handle mechanism of an example TEE probe that incorporates timing ladders according to at least one embodiment of the present disclosure.
**Figure 9** is an isometric view of a portion of the mid-handle mechanism of an example TEE probe that incorporates pulley cables according to at least one embodiment of the present disclosure.
**Figure 10** is an isometric view of a portion of the mid-handle mechanism of an example TEE probe that incorporates pulley cables according to at least one embodiment of the present disclosure.
**Figure 11** is an isometric view of a portion of the mid-handle mechanism of an example TEE probe that incorporates pulley cables according to at least one embodiment of the present disclosure.
**Figure 12** is an isometric view of a portion of the mid-handle mechanism of an example TEE probe that incorporates pulley cables according to at least one embodiment of the present disclosure.
**Figure 13** is an isometric view of a portion of the mid-handle mechanism of an example TEE probe that incorporates timing belts according to at least one embodiment of the present disclosure.
**Figure 14a** is an isometric view of a portion of the mid-handle mechanism of a conventional TEE probe that uses a rack and pinion mechanism to transfer motion from the control wheels to the pull cables.
**Figure 14b** is an isometric view of a portion of the mid-handle mechanism of an example TEE probe that incorporates timing belts according to at least one embodiment of the present disclosure.
**Figure 15** is an isometric view of a portion of the mid-handle mechanism of an example TEE probe that incorporates timing belts according to at least one embodiment of the present disclosure.
**Figure 16** is an isometric view of a tape drive according to at least one embodiment of the present disclosure.
**Figure 17** is an isometric view of a portion of the mid-handle mechanism of an example TEE probe that incorporates timing belts according to at least one embodiment of the present disclosure.
**Figure 18** is a detail view of an example pull cable attachment assembly according to at least one embodiment of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

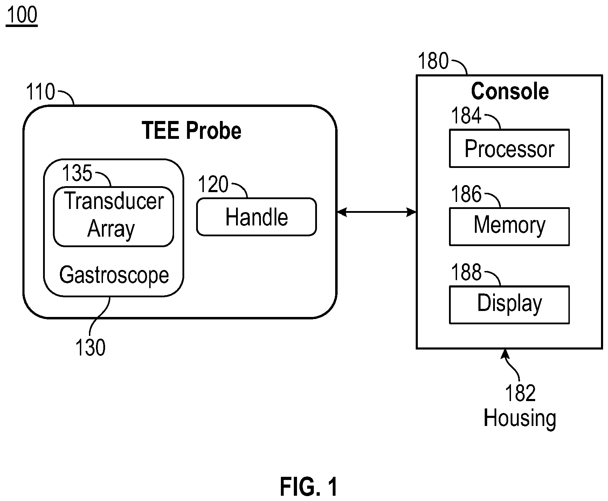

**Figure 1** depicts a diagrammatic view of an ultrasonic imaging system 100 according to an embodiment of the present disclosure. In some embodiments, the ultrasonic imaging system includes a TEE probe. Referring to the embodiment of Figure 1, the TEE probe 110 includes a gastroscope 130 in communication with a handle 120, and the handle 120 is in communication with a console 180, that may include a housing 182, processor 184, memory 186, and display 188. The processor 184, memory 186 and display 188 may be integral to the console or may be removable or otherwise separate. The memory may include either or both of random access memory (RAM) and long-term storage including but not limited to a hard disk drive (HDD), solid state disk (SSD), CD-ROM, flash drive, or other related technologies.

An ultrasound transducer array 135 disposed at a distal portion of the gastroscope 130 may obtain ultrasonic imaging data and transmit the ultrasonic imaging data to the console 180, via communication lines extending through the gastroscope 130, the handle 120, and a cable extending between the TEE probe 110 and the console 180. Additionally, the gastroscope 130 and the ultrasound transducer array 135 may receive control data from the console 180 via the handle 120, or may receive control inputs directly from control surfaces on the handle 120, to control various aspects of a TEE scan, such as one or more firing sequences of an ultrasonic transducer array, and the movement or flexion of the distal portion of the gastroscope 130.

In some embodiments, the gastroscope 130 transmits the ultrasonic imaging data to the console 180 through the handle 120. The handle 120 may also include electronic components to modulate or process the ultrasonic imaging data being transmitted to the console 180.

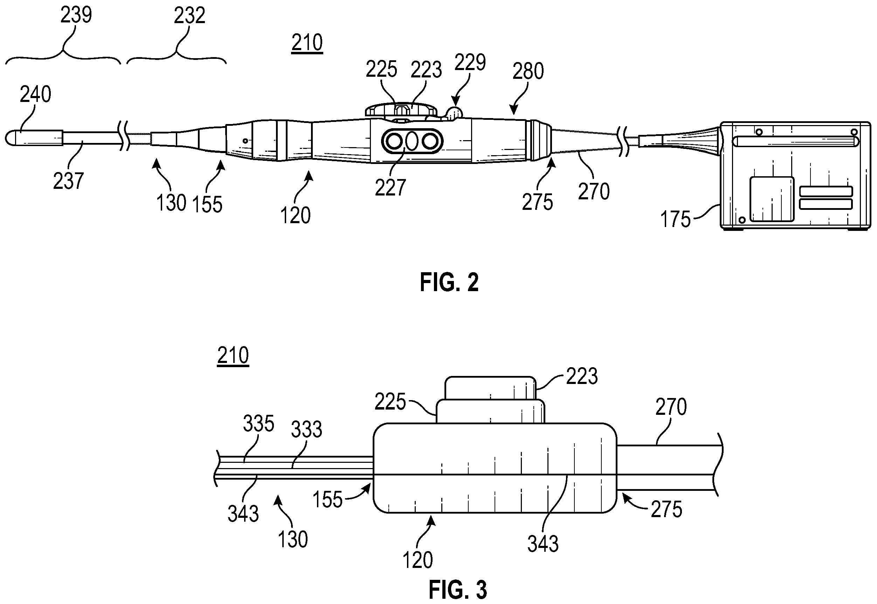

**Figure 2** is a side elevation view of a TEE probe, and depicts a TEE probe 210, including a gastroscope 130, a handle 120, a console interface cable 270, and a connector 175. A TEE handle 120 is permanently connected to the gastroscope at a probe interface connection 155 through which continuous pull cables, power wires, signal wires, and other supporting hardware may be routed. The handle 120 is also permanently connected to the console interface cable 270 at a console interface cable connection 275, through which continuous power wires, signal wires, and other supporting hardware may be routed. A distal end of the cable 270 is coupled to the handle 120 at the console interface cable connection 270. A proximal end of the cable 270 is coupled to the connector 175. The connector 175 can be removably, mechanically coupled to the housing 182 of the console 180 to facilitate signal communication between the console 180, handle 120, and/or the transducer array 135. The connector 175 can be referenced as a patient interface module (PIM) in some embodiments. The TEE probe 210 comprises an integral unit wherein the gastroscope 130 and console interface cable 270 are permanently fixed to the handle 20 at the permanent connections 155, 275 by adhesives, screws, welds, or other attachments which are not readily separated by a physician or user.

The term "permanent" may be used to refer broadly to a mode of coupling or attachment that is not configured or designed for regular detachment and reattachment. Thus, although two coupled components can be physically separated, this disclosure may refer to the coupling or attachment as permanent when the components are not detached in the ordinary medical usage of the device. The terms coupled, coupling, coupled to, or connected, are used to refer broadly to any combination, attachment, or connection. This disclosure may refer to two components as coupled, even though they are permanently fixed to one another, or integrally formed as a single unit. Coupled, as used in this disclosure, contemplates direct and indirect modes of coupling or attachment, and modes of coupling or attachment that are removable. Two components may be referred to as coupled to one another even though they do not directly contact one another, or there are one or more conjoining components between the two components. Removable, separable, or detachable may be used to refer to a mode of coupling or attachment that is configured to be separated and reattached in the normal course of use of a device or system, such as before, during, or after a medical procedure.

The gastroscope 130 includes an elongate body 237, a proximal portion 232, and a distal portion 239 comprising a probe tip 240. The probe tip 240 can include the ultrasound transducer array 135 (Figure 1). The elongate body 237 may be flexible such that it may be guided through the esophagus of the patient by operation of the movement controls 223, 225 coupled to the handle 120. The movement controls 223 and 225 are control inputs to the gastroscope, and may take the form of wheels, knobs, dials, sliders, levers, or other related mechanical inputs. The device's response to actuation of the control inputs may enable a physician to control movement of the distal portion 239 of the gastroscope 130. Controlling movement of the distal portion 239 of the gastroscope 130 can aid the physician to guide the gastroscope 130 through the physiology of the patient to the desired imaging location. A rigid or fixed configuration of a gastroscope could injure the patient, and may be unsuitable for ultrasonic imaging, which demands accurate placement and sufficient contact with the patient's physiology to obtain ultrasonic images. The exemplary handle 120 of Figure 2 also includes a beam-steering control 227 that may affect the geometry of an ultrasonic imaging beam.

**Figure 3** is a diagrammatic view of the handle portion of the TEE probe of Figure 2. The gastroscope 130 includes first and second pull cables 333, 335 that are mechanically connected to first and second movement controls 223, 225 coupled to the handle 120. Such a configuration may enable a physician to control movement of the distal portion 239 of the gastroscope 130. Controlling movement of the distal portion 239 of the gastroscope 130 can aid the physician to guide the gastroscope 130 through the physiology of the patient to the desired imaging location, as ultrasonic imaging demands accurate placement and sufficient contact with the patient's physiology to obtain ultrasonic images. Thus, pull cables 333, 335 and flexible components aid the physician in obtaining ultrasonic images of the patient's organs. Because the first and second pull cables 333, 335 are mechanically connected to the first and second movement controls 223, 225, a physician manipulating the first and second movement controls 223, 225 may receive tactile feedback from the distal portion 239 of the gastroscope 130 when the distal portion 239 presses against the walls of the esophagus. The exemplary handle of Figure 3 also includes transducer wiring 343 to convey signals from the gastroscope 130 to the console 180.

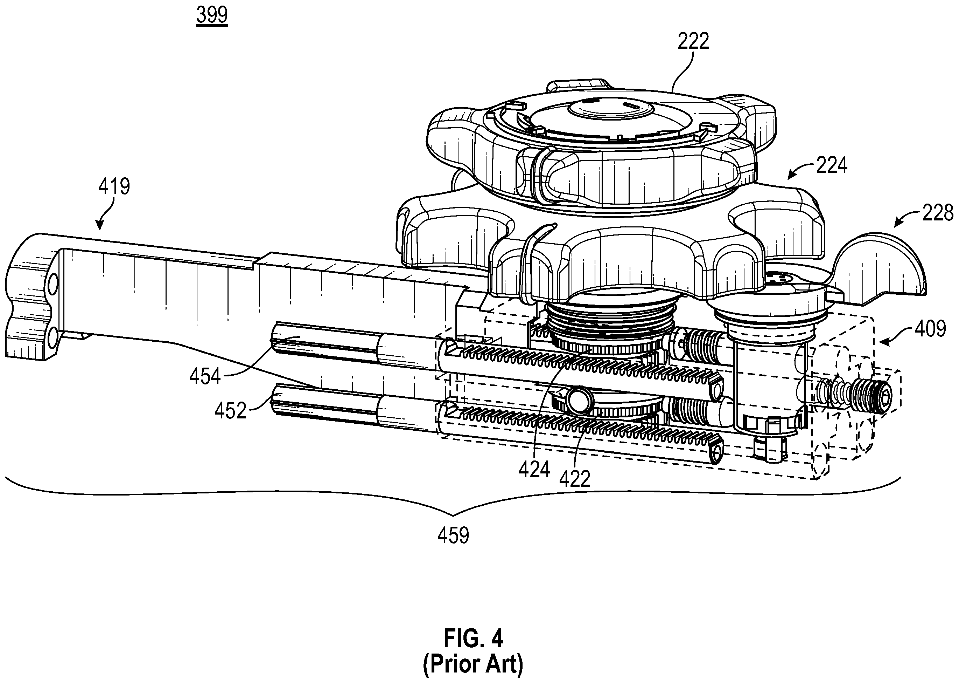

**Figure 4** depicts a translucent isometric view of the interior mechanism 399 of an example TEE mid-handle assembly of a conventional TEE probe. The first movement control 222 and second movement control 224 create rotation in corresponding first pinion shaft 422 and second pinion shaft 424, which then create linear motion in first rack 452 and second rack 454 via a rack-and-pinion mechanism. An additional first rack 452 and second rack 454 are on the far side of the visible mechanism and operate in conjunction with the visible racks 452 and 454 according to the same principle. The first racks 452 and second racks 454 are coupled to first and second pull cables to extend or contract them, and thus to control movement of the distal portion of the gastroscope.

The mechanism thus described is housed within a containment frame 459 that includes a gear housing 409 and a distal isolator 419. The mid-handle mechanism also includes a brake switch 228 that may be engaged to apply resistance to the rotation of movement controls 222 and 224. The rack and pinion mechanism requires tight manufacturing tolerances in order to function correctly, and may therefore be machined from steel, fully hardened beryllium copper, or other metals. The brake switch 228 may also be a multi-part assembly of four or more parts. The distal isolator may be machined from hard polymer materials such as 20% glass-filled polycarbonate. The rack frame or gear housing requires multiple apertures, through-holes, and threaded holes in order to accommodate other parts in the mid-handle assembly 399, and these features may require rather strict manufacturing tolerances. The gear housing may therefore be machined from metals such as nickel-plated 6061-T6 aluminum.

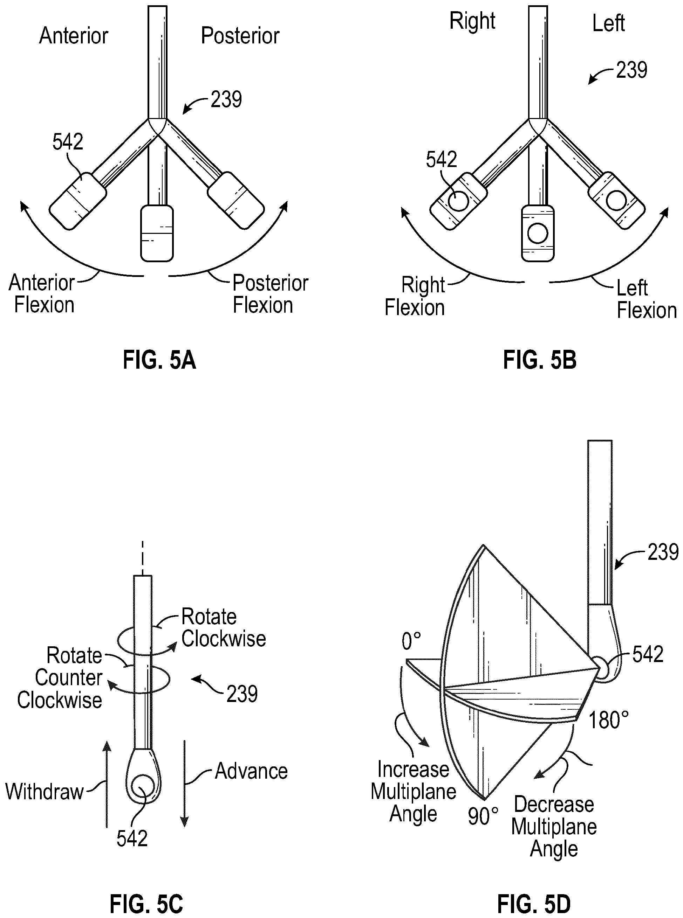

**Figure 5A** is a side elevation view of a lateral side of a distal portion 239 of a TEE probe gastroscope 130, illustrating various modes of anterior and posterior flexion of the distal portion 239, which includes an imaging element 542. The anterior/posterior flexion and right/left flexion may be accomplished by retracting and releasing one or more pull cables corresponding to one or more modes of movement. In some embodiments, the physician may control the anterior or posterior flexion of the distal portion 239 of the gastroscope 130 by retracting or releasing the first pull cable 333. As the first pull cable 333 may comprise a plurality of individual cables or wires, controlling the anterior or posterior flexion of the distal portion of the gastroscope 130 may include retracting one of the individual wires of the first pull cable 333, while leaving another of the individual wires of the first pull cable 333 static.

**Figure 5B** is an elevation view of a front side of the distal portion 239 of the TEE probe gastroscope 130 of Figure 5A, illustrating left and right flexion of the distal portion 239, which includes imaging element 542. The right/left flexion may be accomplished by retracting and releasing one or more pull cables corresponding to one or more modes of movement. In some embodiments, the physician may control the anterior or posterior flexion of the distal portion 239 of the gastroscope 130 by retracting or releasing the second pull cable 335. As the second pull cable 335 may comprise a plurality of individual cables or wires, controlling the left and right flexion of the distal portion of the gastroscope 130 may include retracting one of the individual wires of the second pull cable 335, while leaving another of the individual wires of the second pull cable 335 static.

**Figure 5C** is a side elevation view of the front side of the distal portion of the TEE probe of Figure 5A, illustrating modes of physical movement including counterclockwise rotation, clockwise rotation, advanced and withdrawn positioning of the distal portion. Such motions can be achieved by manually adjusting the gastroscope 130, such as by rotating the handle to rotate the gastroscope 130 within the esophagus of the patient. A physician may also advance or withdraw the distal portion 239 of the gastroscope 130 within the esophagus of the patient by advancing or withdrawing the handle toward or away from the patient's esophagus.

**Figure 5D** is an isometric view of the distal portion of the TEE probe of Figure 5A, illustrating increasing and decreasing multi-plane angles of an ultrasonic transducer. The electronic modes of movement depicted in Figure 5D may not require physical manipulation of the distal portion of the gastroscope, such as the modes of movement depicted in Figures 5A-5C, but may be accomplished by controlling one or more electronic aspects of an ultrasonic transducer array or imaging element 542, such as the timing of firing and receive sequences. This may be initiated via electronic commands from the console 180 or from a beam-steering control 227 in the handle 120.

Referring generally to Figures 5A-5D, the illustrated various modes of movement and flexion of the distal portion 239 of the gastroscope 130 may be controlled by one or more user input selectors of the handle (e.g., 223, 225, Figure 2). For example, in one embodiment, the anterior and posterior flexion of the distal portion 239 of the gastroscope 130 are controlled by a first user input selector 223, while the left and right flexion are controlled by a second user input selector 225. The rotating, advancing, and withdrawal of the distal portion 239 can be achieved by manually rotating, advancing, and withdrawing the handle 120 of the TEE probe 110. The electronic or beam angle movement may be manipulated or adjusted by a third user input selector 227. As described above, the user input selectors 223, 225, and 227 may each comprise at least one of an electronic button, dials, capacitive touch sensors, levers, switches, knobs, joysticks, etc.

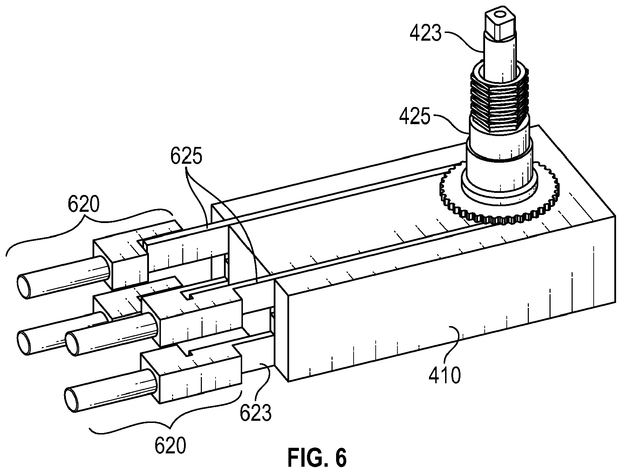

**Figure 6** is an isometric view of a portion of the mid-handle mechanism of an example TEE probe that incorporates friction belts according to at least embodiment of the present disclosure. The rotation of shaft 423 creates a linear motion in friction belt 623, which is attached to pull cables 333 by two pull cable attachment assemblies 620. The pull cable attachment assemblies 620 may comprise clips, clamps, collars, collets, sleeves, thimbles, swivels, swages, hooks, eye fittings, clevis fittings, stud fittings, ball and dome fittings, turnbuckles, tensioners, and other related mechanical fittings suitable for attaching to a pull cable, plus any appropriate housings, fasteners, and adaptors. The rotation of shaft 425 similarly creates a linear motion in fiction belt 625 which is attached to pull cables 335 by another pair of pull cable attachment assemblies 620. Thus, the rotation of the shafts controls movements of the distal end 239 of the gastroscope 130. Also pictured is the gear housing 410. In an example, the friction belts 623 and 625 are nonconductive.

In an example, a portion of the pinion shafts 423 and 425 is internal to the gear housing 410, and the portion of the shafts 423 and 425 that is external to the gear housing 410 is internal to the control knobs 223 and 225, such that the shafts 423 and 425 are minimally exposed to view or not exposed to view in a fully assembled device.

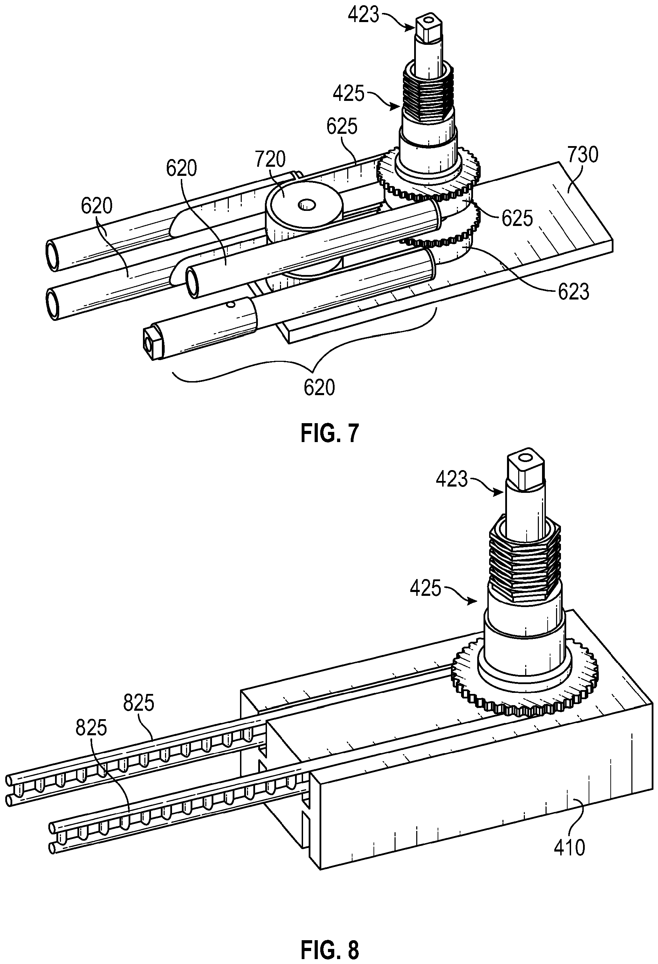

**Figure 7** is an isometric view of a portion of the mid-handle mechanism of an example TEE probe that incorporates friction belts according to at least embodiment of the present disclosure. The wrapping of belts 623 and 625 around corresponding shafts 423 and 435 is visible. This embodiment includes a different type of pull cable attachment assembly 620, and also includes an idler 720 to assist in the linear actuation of belts 623 and 625, such that the belts 623 and 625 wrap around the pinion shafts and past the idler to give a push-pull action. The belts 623 and 625 may be made of elastomeric compounds molded over Kevlar, nylon, or metal reinforcement members to reduce stretch and increase life. Pull cable attachment assemblies 620 may be molded directly onto the friction belts. Also pictured is a base 730.

In the example shown in the figure, the belts 623 and 625 are wrapped 180 degrees around the shafts 423 and 425 (i.e., partially positioned around a perimeter or circumference of the shaft), such that the two ends of each belt move in opposite directions when the shaft is rotated. It should be understood that other degrees of wrapping are possible, including those that yield substantially opposite motion (e.g., 170 or 190 degrees), those that yield substantially similar motion (e.g., 10 degrees, 0 degrees, or 370 degrees), and otherwise.

**Figure 8** is an isometric view of a portion of the mid-handle mechanism of an example TEE probe that incorporates timing ladders or timing ladder belts according to at least embodiment of the present disclosure. In a manner analogous to Figure 6, timing ladder 825 is coupled to pinion shaft 425 inside gear housing 410 such that rotation of shaft 425 creates linear motion in timing ladder 825. However, unlike the friction belts 623 and 625 of Figure 6, the timing ladder 825 includes features that may engage the teeth of a gear or pinion, such that friction is not required in order for the ladder to hold its position. The ladder belt 825 wraps around the corresponding pinion shaft 425 to create a push-pull action. Ladder belt 825 may be made from elastomeric compounds molded over steel, Kevlar, or nylon reinforcement members to reduce stretch and increase life. An additional ladder belt may be added to the mid-handle mechanism to wrap around and engage with pinion shaft 423, such that one ladder belt may control left/right flexion of the distal portion 239 and probe tip 240, and the other ladder belt may control anterior/posterior flexion of the distal portion 239 and probe tip 240.

Pinion shaft 423 is also pictured. In an example, the timing ladder 825 is nonconductive. Pull cable attachment assembly 620 may be mechanically attached to and/or molded into the timing ladder 825.

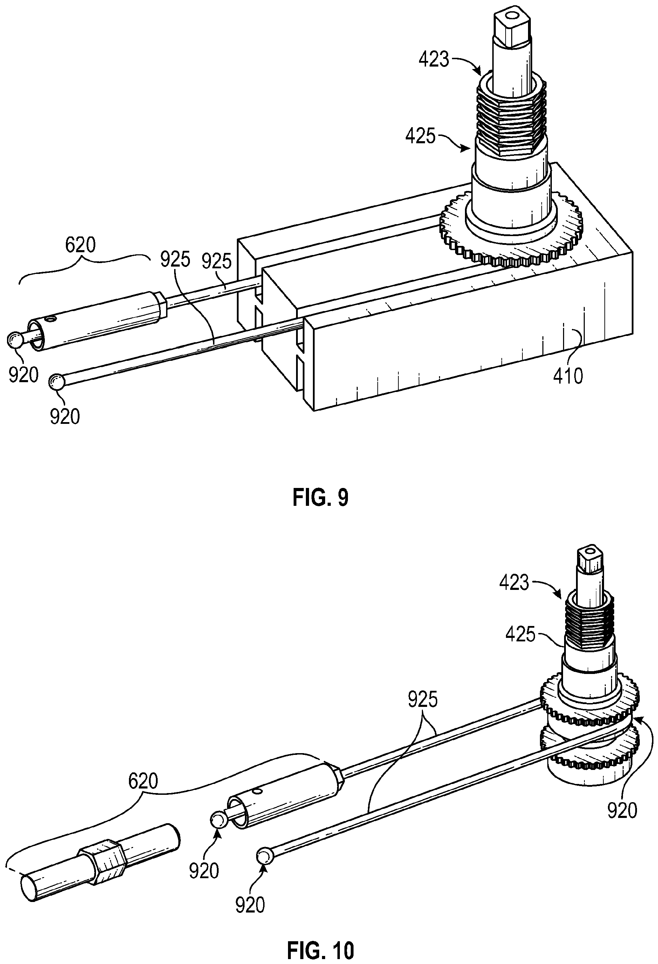

**Figure 9** is an isometric view of a portion of the mid-handle mechanism of an example TEE probe that incorporates pulley cables according to at least one embodiment of the present disclosure. In a manner analogous to Figures 6 and 8, pulley cable 925 is coupled to shaft 425 inside gear housing 410 such that rotation of shaft 425 creates linear motion in pulley cable 925. Shaft 423 is also pictured. The pull cable 335 attach to pulley cable 925 via pull cable attachment assemblies 620, aided by swaged balls 920 at the ends of pulley cable 925.

In an example, the pulley cable 925 is made from a non-metallic, non-conductive rope such as Kevlar or similar material. In another example, the pulley cable 925 is made from a polymer-overcoated metal cable. A pull cable attachment device 620 such as a threaded collet can be held captive on either end of the Kevlar ropes and used to attach to and adjust final cable tension. An additional pulley cable may be added to the mid-handle mechanism to wrap around and engage with shaft 423 in the same manner, such that one of the pulley cables may control left-right deflection of the distal portion 239 and probe tip 240, and the other pulley cable may control anterior/posterior deflection of the distal portion 239 and probe tip 240.

**Figure 10** is an isometric view of a portion of the mid-handle mechanism of an example TEE probe that incorporates pulley cables according to at least embodiment of the present disclosure. In this view, the gear housing 410 is not shown, such that the association of pulley cable 925 with shaft 425 can be seen. An additional swaged ball 920 in the middle of cable 925 keeps it from sliding around shaft 425. Multiple balls may be swaged onto the Kevlar rope at timed locations if control knob rotates more than 180 degrees. Shaft 423 is also pictured.

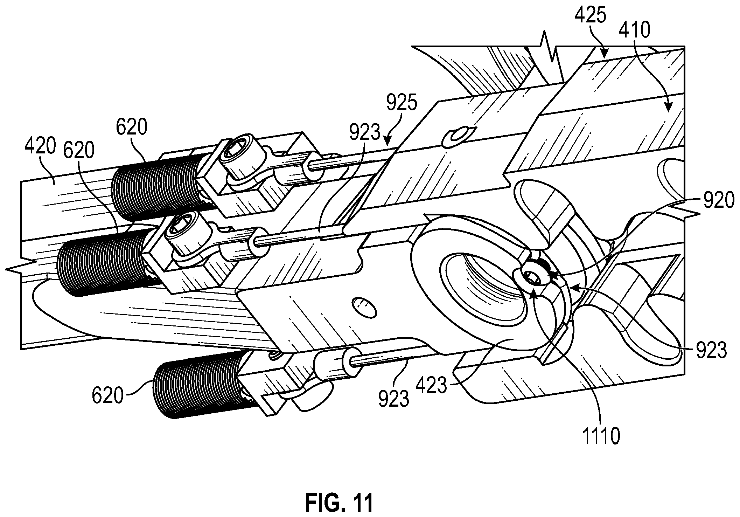

**Figure 11** is an isometric view of a portion of the mid-handle mechanism of an example TEE probe that incorporates pulley cables according to at least embodiment of the present disclosure. This view provides detail on a swaged ball or screw attachment 920 that is provided in the middle of pulley cable 923 and fits into a recess 1110 in shaft 423 to keep pulley cable 923 from slipping with regard to shaft 423. Also pictured are pulley cable 925, gear housing 410, three pull cable attachment assemblies 620, and the distal isolator 420.

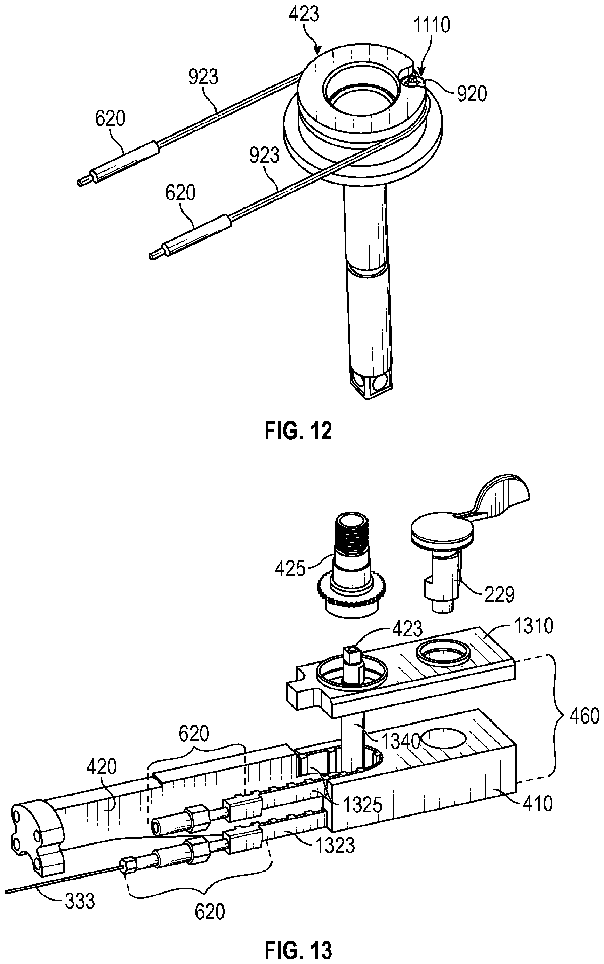

**Figure 12** is an isometric view of a portion of the mid-handle mechanism of an example TEE probe that incorporates pulley cables according to at least embodiment of the present disclosure. This view includes shaft 423, which is inverted 180 degrees from its orientation in Figure 11. Pulley cable 923 wraps around shaft 423 and anchors to it with a swaged ball or screw attachment 920. Pulley cable 923 attaches to pull cables 333 via the pull cable attachment assemblies 620. The use of an anchored pulley cable allows the shaft 423 to be a simple pulley shaft rather than a toothed gear shaft or pinion shaft, thus reducing the material requirements, manufacturing tolerances, and geometric complexity of the part.

**Figure 13** is an isometric, exploded view of a portion of the mid-handle mechanism of an example TEE probe that incorporates timing belts according to at least embodiment of the present disclosure. In a manner analogous to the timing ladders of Figure 8, timing belts 1323 and 1325 engage with pinion shafts 423 and 425 such that a rotation of either shaft causes a linear motion in the corresponding timing belt, which then creates linear motion in the pull cable attachment assembly 620 to move the corresponding pull cable 333 or 335. Also pictured is a brake switch 229 that, due to its 3D shape, applies resistance to the rotation of pinion shafts 423 and 425 when the brake switch 229 is rotated into its engaged position, and permits shafts 423 and 425 to rotate when brake switch 229 is rotated into its disengaged position.

In this embodiment, the containment frame 460 comprises a distal isolator 420, gear housing 410, and header 1310. Also pictured is a floating, nonrotating interior shaft 1340 that sits between the inner pinion shaft 423 and outer pinion shaft 425 for 2 way articulation. The purpose of this shaft 1340 is to isolate any motion that could otherwise be transferred from either inner to outer or outer to inner control shafts during operation; this may eliminate or nearly eliminate cross-coupling of motion between the two control knobs. In an example, the floating shaft 1340 is mechanically constrained from rotating by connection to internal frame. The floating shaft 1340 may be formed as part of the gear housing 410 or base 730.

In an example, parts of the shafts 423, 425, and 1340 are located interior to the containment frame 460, and the parts that are exterior to the containment frame 460 are interior to the control knobs 223 and 225, such that in a fully assembled device they are minimally exposed or not exposed to view. In an example, the timing belts are nonconductive, and may be made of elastomeric compounds molded over Kevlar, nylon, or metal reinforcement members to reduce stretch and increase life. Pull cable attachment assemblies 620 may be molded directly onto the timing belts, although the form and materials of such interface assemblies are specific to each embodiment and may include additional features such as screw tension adjustments.

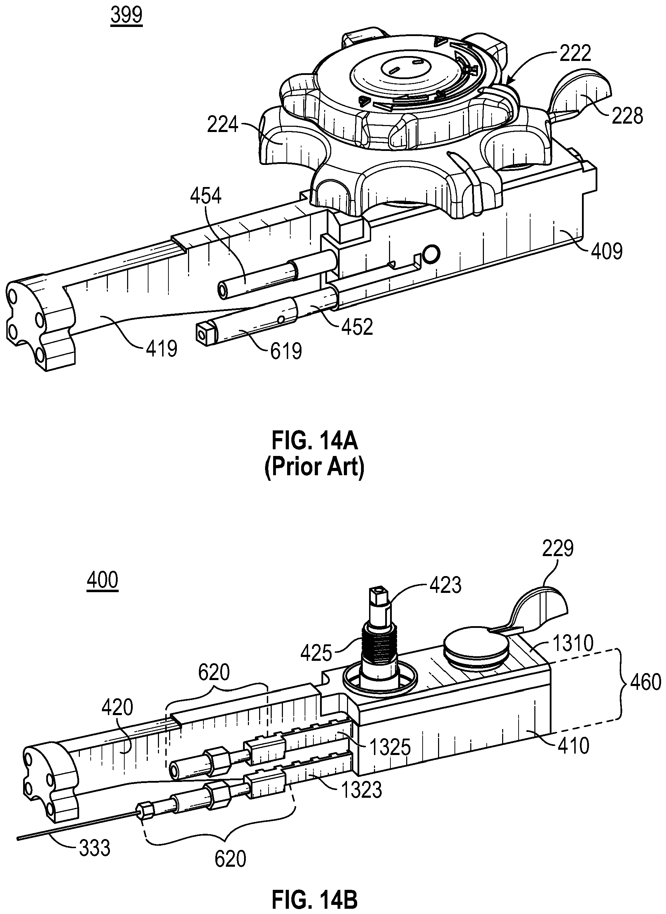

**Figure 14a** is an isometric view of a portion of the mid-handle mechanism 399 of a conventional TEE probe that uses a rack and pinion mechanism to transfer motion from the control wheels to the pull cables. Visible are the control wheels 222 and 224, brake switch 228, gear housing 409, distal isolator 419, rack actuators 452 and 454, and pull cable attachment assembly 619. The rack and pinion mechanism requires tight manufacturing tolerances in order to function correctly, and may therefore be machined from steel, fully hardened beryllium copper, or other metals. The brake switch 228 may also be a multi-part assembly of four or more parts. The distal isolator may be machined from hard polymer materials such as glass-filled polycarbonate. The rack frame or gear housing provides a rather complex shape that may be machined from metals such as 6061-T6 aluminum.

**Figure 14b** is an isometric view of a portion of the mid-handle mechanism 400 of an example TEE probe that incorporates timing belts according to at least one embodiment of the present disclosure. Visible are the brake switch 229, pull cable 333, gear housing 410, pinion shafts 423 and 425, pull cable attachment assembly 620, header 1310, and timing belts 1323 and 1325. A reader of ordinary skill in the art will understand that the assembly of Figure 14b has similar overall size and shape to that of Figure 14a, and is operated in the same manner, but in accordance with the disclosure of Figures 6-13 it has a greatly simplified internal structure vs. that of Figures 4 and 14a, and includes a smaller overall number of parts. For example, the gear housing 410 and distal isolator 420 of the containment frame 460 may be formed as a single piece, with fewer openings, through-holes, threaded holes, and other complex features. The brake switch assembly may also be monolithic (i.e., formed as a single piece). The timing belts 1323 and 1325 each replace multiple components of the rack-and-pinion system, and may require significantly looser manufacturing tolerances such that, for example, the brake switch 229, pinion shafts 423 and 425, and containment frame 460 may be made rapidly and inexpensively from cast-molded or injection-molded plastic, rather than machined from steel, aluminum, fully hardened beryllium copper, glass-filled polycarbonate, or other metals or polymers requiring substantially greater time and cost. Depending on the implementation, some of these components may continue to be made of metal in areas where wear, fatigue, or stretch are concerning. For example, in some embodiments, the brake switch and pinion shafts are machined from metal.

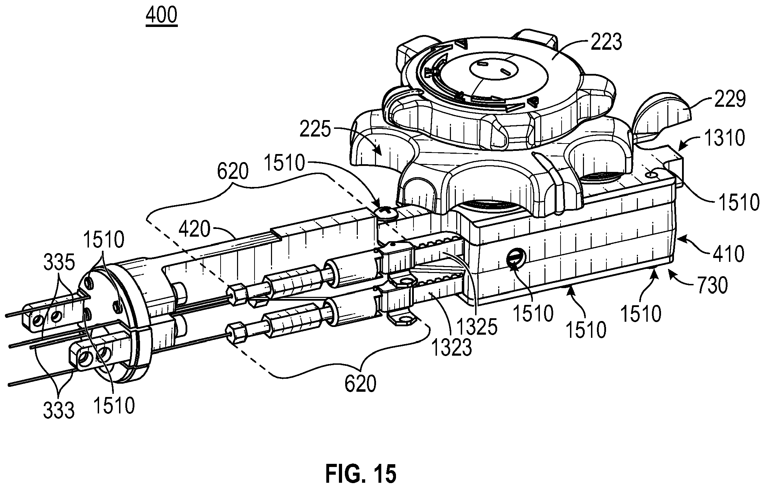

**Figure 15** is an isometric view of a portion of the mid-handle mechanism 400 of an example TEE probe that incorporates timing belts according to at least one embodiment of the present disclosure. This embodiment includes an assembly that may be manufactured and assembled more easily than the current TEE probe mid-handle assembly of Figures 4 and 14a. Visible in this view are the control wheels 223 and 225, brake switch 229, pull cables 333 and 335, gear housing 410, distal isolator 420, pull cable attachment assemblies 620, base 730, header 1310, timing belts 1323 and 1325, and a number of fasteners 1510.

In an example, parts of the shafts 423, 425, and 1340 are located interior to the containment frame 460, and the parts that are exterior to the containment frame 460 are interior to the control knobs 223 and 225, such that in a fully assembled device they are minimally exposed or not exposed to view.

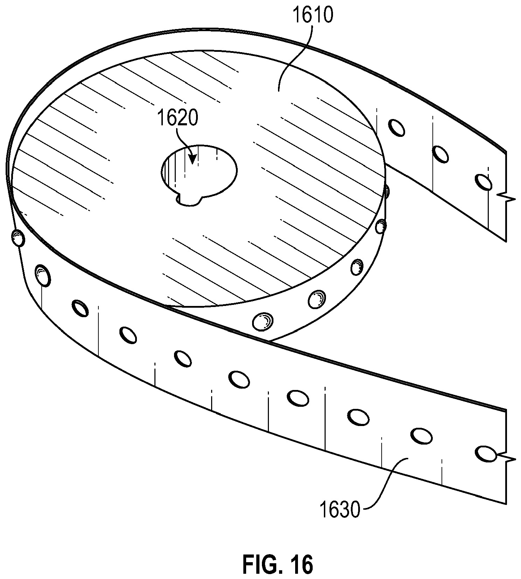

**Figure 16** is an isometric view of a tape drive according to at least one embodiment of the present disclosure. In a manner analogous to the timing belts and timing ladders already disclosed, a tape drive can convert the rotational motion of a shafted gear into linear motion of a tape. As with a timing belt or timing ladder, the tape incorporates features that may engage with teeth or other features of a gear such that the tape does not slip with respect to the gear and does not require friction in order to rotate along with the gear. Visible in this view are a gear 1610, drive tape 1630, and a socket 1620 through which a shaft may be placed. The incorporation of a tape drive within the gear housing, in place of pulley cables, timing ladders, timing belts, or friction belts will be understood by a person of ordinary skill in the art, and may for example offer one or more of increased material strength, decreased component stretch, comparable manufacturing efficiency, and improved reliability and lifespan vs. the friction belt, timing ladder, timing belt, and pulley cable designs described hereinabove. In an example, the drive tape 1630 is nonconductive.

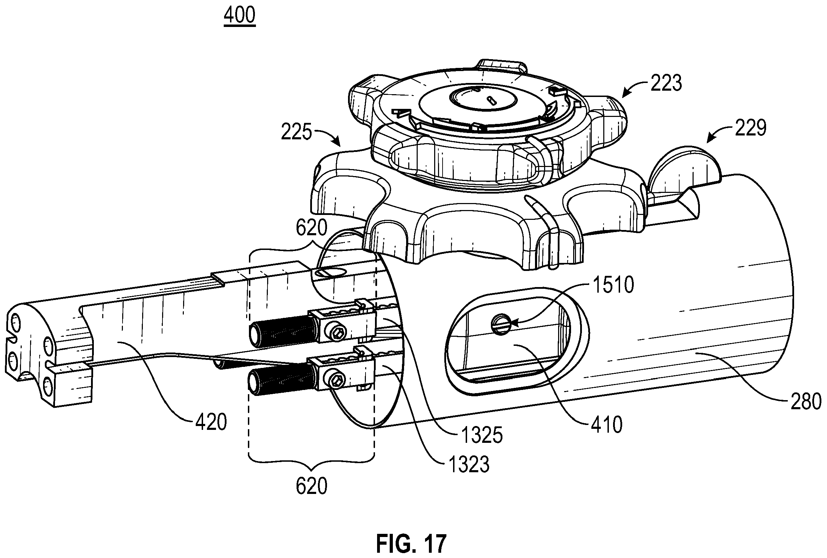

**Figure 17** is an isometric view of a portion of the mid-handle mechanism 400 of an example TEE probe that incorporates timing belts according to at least one embodiment of the present disclosure. This figure is analogous to Figure 15, except that it includes an outer handle casing 280 as shown for example in Figure 2, in order to more fully convey the relationship of the mid-handle mechanism 400 to the whole of the handle 120. Also visible are the control wheels 223 and 225, brake switch 229, gear housing 410, distal isolator 420, pull cable attachment assemblies 620, and timing belts 1323 and 1325.

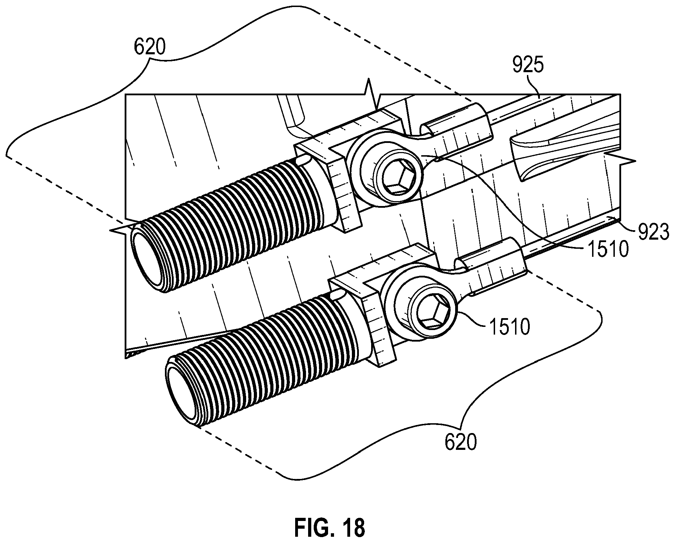

**Figure 18** is a detail view of an example pull cable attachment assembly according to at least one embodiment of the present disclosure. Visible are two pull cable attachment assemblies 620, along with pulley cables 923 and 925, and fasteners 1510 to help connect the pulley cables 923 and 925 to the pull cable attachment assemblies 620.

After becoming familiar with the teachings herein, persons of ordinary skill in the art will recognize that the disclosed TEE probe mid-handle assembly provides a substantially less complicated assembly that is naturally electrically isolated, and is easier and less costly to build in production, than existing TEE probe mid-handle assemblies.

Persons skilled in the art will also recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. A transesophageal echocardiography probe, comprising:
a containment frame (460);
a first control input (225) located external to the containment frame;
a first shaft (425) located internal to the containment frame and coupled to the first control input;
a first elongated flexible member (625, 825, 925, 1325, 1630) comprising a first end and a second end, wherein the first elongated flexible member is wrapped around the first shaft;
a first pull cable attachment (620) coupled to the first end of the first elongated flexible member;
a second pull cable attachment (620) coupled to the second end of the first elongated flexible member;
a first pull cable (335) attached to the first pull cable attachment; and
a second pull cable (335) attached to the second pull cable attachment, wherein, when the first control input is actuated, the first shaft is rotated such that the first and second ends of the first elongated flexible member move linearly, causing one of the first or second pull cable to move in a first direction and causing the other of the first or second pull cable to move in an opposite, second direction, and
wherein a distal portion (239) of a gastroscope (130) coupled to the first and second pull cables is flexed according to movements of the first and second pull cables.

2. The transesophageal echocardiography probe of claim 1, further comprising:
a second control input located external to the containment frame;
a second shaft located internal to the containment frame and coupled to the second control input;
a second elongated flexible member comprising a first end and a second end, wherein the second elongated flexible member is wrapped around the second shaft;
a third pull cable attachment coupled to the first end of the second elongated flexible member;
a fourth pull cable attachment coupled to the second end of the second elongated flexible member;
a third pull cable attached to the third pull cable attachment; and
a fourth pull cable attached to the fourth pull cable attachment,
wherein, when the second control input is rotated, the second shaft is rotated such that the first and second ends of the second elongated flexible member move linearly, causing one of the third or fourth pull cable to move in the first direction, and causing the other of the third or fourth pull cable to move in the opposite, second direction, and
wherein the distal portion of the gastroscope coupled to the third and fourth pull cables is flexed according to movements of the third and fourth pull cables.

3. The transesophageal echocardiography probe of claim 2, further comprising a monolithic brake switch that, when rotated into a first position, applies a resistance to rotation of the first and second shafts and, when rotated into a second position, permits the first and second shafts to rotate without the resistance.

4. The transesophageal echocardiography probe of claim 3, wherein at least one of the control inputs, shafts, elongated flexible members, brake switch, or containment frame is nonconductive.

5. The transesophageal echocardiography probe of claim 4, wherein at least one of the control inputs, shafts, brake switch, or containment frame is made of molded plastic.

6. The transesophageal echocardiography probe of claim 2, wherein the first and second elongated flexible members are made of elastomeric compounds molded over nylon reinforcement members.

7. The transesophageal echocardiography probe of claim 2, wherein the first control input directs anterior and posterior flexion of the distal portion of the gastroscope, and the second control input directs left/right flexion of the gastroscope.

8. The transesophageal echocardiography probe of claim 2, wherein the first and second elongated flexible members are friction belts.

9. The transesophageal echocardiography probe of claim 2, wherein the first and second shafts comprise gears or pinions, and wherein the first and second elongated flexible members are timing ladder belts interfacing with the gears or pinions,
or
wherein the first and second shafts comprise gears or pinions, and wherein the first and second elongated flexible members are timing belts interfacing with the gears or pinions,
or
wherein the first and second shafts comprise gears or pinions, and wherein the first and second elongated flexible members are drive tapes interfacing with the gears or pinions.

10. The transesophageal echocardiography probe of claim 2, wherein the first shaft and second shaft each comprise a socket, and wherein the first elongated flexible member is a pulley cable anchored to the first shaft by a swaged ball or screw attachment fitting into the socket in the first shaft, and wherein the second elongated flexible member is a pulley cable anchored to the second shaft by a swaged ball or screw attachment fitting into the socket in the second shaft.

11. The transesophageal echocardiography probe of claim 2, wherein the pull cable attachments are formed directly into the elongated flexible members.

12. The transesophageal echocardiography probe of claim 2, wherein the pull cable attachments each include a threaded tensioning mechanism.

13. The transesophageal echocardiography probe of claim 2, further comprising an outer handle casing.

14. The transesophageal echocardiography probe of claim 2, wherein the first and second shafts are coaxial.

15. The transesophageal echocardiography probe of claim 2, further comprising a floating, nonrotating interior shaft affixed to the containment frame and situated between the first and second shafts to limit cross-coupling of motion between the first and second shafts.

## Patentansprüche

1. Transösophageale Echokardiographiesonde, umfassend:
einen Halterahmen (460);
einen ersten Steuereingang (225), der sich außerhalb des Halterahmens befindet;
eine erste Welle (425), die sich innerhalb des Halterahmens befindet und mit dem ersten Steuereingang verbunden ist;
ein erstes längliches flexibles Element (625, 825, 925, 1325, 1630), das ein erstes Ende und ein zweites Ende umfasst, wobei das erste längliche flexible Element um die erste Welle gewickelt ist;
eine erste Zugkabelbefestigung (620), die mit dem ersten Ende des ersten länglichen flexiblen Elements verbunden ist;
eine zweite Zugkabelbefestigung (620), die mit dem zweiten Ende des ersten länglichen flexiblen Elements verbunden ist;
ein erstes Zugkabel (335), das an der ersten Zugkabelbefestigung befestigt ist; und
ein zweites Zugkabel (335), das an der zweiten Zugkabelbefestigung befestigt ist,
wobei, wenn die erste Steuereingabe betätigt wird, die erste Welle so gedreht wird, dass sich die ersten und zweiten Enden des ersten länglichen flexiblen Elements linear bewegen, wodurch sich eines des ersten oder zweiten Zugkabels in eine erste Richtung bewegt und das andere des ersten oder zweiten Zugkabels sich in eine entgegengesetzte, zweite Richtung bewegt, und
wobei ein distaler Abschnitt (239) eines Gastroskops (130), der mit den ersten und zweiten Zugkabeln gekoppelt ist, entsprechend den Bewegungen der ersten und zweiten Zugkabel gebogen wird.

2. Transösophageale Echokardiographiesonde nach Anspruch 1, weiterhin umfassend:
einen zweiten Steuereingang, der sich außerhalb des Halterahmens befindet;
eine zweite Welle, die sich innerhalb des Halterahmens befindet und mit dem zweiten Steuereingang gekoppelt ist;
ein zweites längliches flexibles Element, das ein erstes Ende und ein zweites Ende umfasst, wobei das zweite längliche flexible Element um die zweite Welle gewickelt ist;
eine dritte Zugkabelbefestigung, die mit dem ersten Ende des zweiten länglichen flexiblen Elements verbunden ist;
eine vierte Zugkabelbefestigung, die mit dem zweiten Ende des zweiten länglichen flexiblen Elements verbunden ist;
ein drittes Zugkabel, das an der dritten Zugkabelbefestigung befestigt ist; und
ein viertes Zugkabel, das an der vierten Zugkabelbefestigung befestigt ist,
wobei, wenn der zweite Steuereingang gedreht wird, die zweite Welle so gedreht wird, dass sich die ersten und zweiten Enden des zweiten länglichen flexiblen Elements linear bewegen, wodurch sich eines des dritten oder vierten Zugkabels in die erste Richtung bewegt und das andere des dritten oder vierten Zugkabels sich in die entgegengesetzte, zweite Richtung bewegt, und
wobei der distale Abschnitt des Gastroskops, der mit dem dritten und vierten Zugkabel gekoppelt ist, entsprechend den Bewegungen des dritten und vierten Zugkabels gebogen wird.

3. Transösophageale Echokardiographiesonde nach Anspruch 2, weiterhin umfassend einen monolithischen Bremsschalter, der, wenn er in eine erste Position gedreht wird, einen Widerstand gegen die Drehung der ersten und zweiten Welle ausübt und, wenn er in eine zweite Position gedreht wird, eine Drehung der ersten und zweiten Welle ohne Widerstand ermöglicht.

4. Transösophageale Echokardiographiesonde nach Anspruch 3, wobei mindestens einer der Steuereingänge, Wellen, länglichen flexiblen Elemente, Bremsschalter oder Halterahmen nicht leitend ist.

5. Transösophageale Echokardiographiesonde nach Anspruch 4, wobei mindestens einer der Steuereingänge, Wellen, Bremsschalter oder Halterahmen aus geformtem Kunststoff besteht.

6. Transösophageale Echokardiographiesonde nach Anspruch 2, wobei die ersten und zweiten länglichen flexiblen Elemente aus über Nylonverstärkungselementen geformten Elastomerverbindungen bestehen.

7. Transösophageale Echokardiographiesonde nach Anspruch 2, wobei der erste Steuereingang die vordere und hintere Beugung des distalen Abschnitts des Gastroskops steuert und der zweite Steuereingang die Links-/Rechtsbeugung des Gastroskops steuert.

8. Transösophageale Echokardiographiesonde nach Anspruch 2, wobei das erste und das zweite längliche flexible Element Reibungsriemen sind.

9. Transösophageale Echokardiographiesonde nach Anspruch 2, wobei die ersten und zweiten Wellen Zahnräder oder Ritzel umfassen und wobei die ersten und zweiten länglichen flexiblen Elemente Zahnriemen sind, die mit den Zahnrädern oder Ritzeln in Verbindung stehen,
oder
wobei die ersten und zweiten Wellen Zahnräder oder Ritzel umfassen und wobei die ersten und zweiten länglichen flexiblen Elemente Steuerriemen sind, die mit den Zahnrädern oder Ritzeln in Verbindung stehen,
oder
wobei die ersten und zweiten Wellen Zahnräder oder Ritzel umfassen und wobei die ersten und zweiten länglichen flexiblen Elemente Antriebsbänder sind, die mit den Zahnrädern oder Ritzeln in Verbindung stehen.

10. Transösophageale Echokardiographiesonde nach Anspruch 2, wobei die erste Welle und die zweite Welle jeweils eine Buchse umfassen und wobei das erste längliche flexible Element ein Seilrollenkabel ist, das an der ersten Welle durch eine gestauchte Kugel- oder Schraubenbefestigung verankert ist, die in die Buchse in der ersten Welle passt, und wobei das zweite längliche flexible Element ein Seilrollenkabel ist, das an der zweiten Welle durch eine gestauchte Kugel- oder Schraubenbefestigung verankert ist, die in die Buchse in der zweiten Welle passt.

11. Transösophageale Echokardiographiesonde nach Anspruch 2, wobei die Zugkabelbefestigungen direkt in die länglichen flexiblen Elemente eingeformt sind.

12. Transösophageale Echokardiographiesonde nach Anspruch 2, wobei die Zugkabelbefestigungen jeweils einen Spannmechanismus mit Gewinde umfassen.

13. Transösophageale Echokardiographiesonde nach Anspruch 2, die weiterhin ein äußeres Griffgehäuse umfasst.

14. Transösophageale Echokardiographiesonde nach Anspruch 2, wobei die erste und die zweite Welle koaxial sind.

15. Transösophageale Echokardiographiesonde nach Anspruch 2, die weiterhin eine schwimmende, nicht rotierende Innenwelle umfasst, die am Halterahmen befestigt und zwischen der ersten und der zweiten Welle angeordnet ist, um eine Kreuzkopplung der Bewegung zwischen der ersten und der zweiten Welle zu begrenzen.

## Revendications

1. Sonde d'échocardiographie transœsophagienne, comprenant :
un cadre de confinement (460);
une première entrée de commande (225) située à l'extérieur du cadre de confinement;
un premier arbre (425) situé à l'intérieur du cadre de confinement et couplé à la première entrée de commande;
un premier élément flexible allongé (625, 825, 925, 1325, 1630) comprenant une première extrémité et une deuxième extrémité, où le premier élément flexible allongé est enroulé autour du premier arbre;
une première fixation de câble de traction (620) couplée à la première extrémité du premier élément flexible allongé;
une deuxième fixation de câble de traction (620) couplée à la deuxième extrémité du premier élément flexible allongé;
un premier câble de traction (335) fixé à la première fixation de câble de traction; et
un deuxième câble de traction (335) fixé à la deuxième fixation de câble de traction,
où, lorsque la première entrée de commande est actionnée, le premier arbre tourne de telle sorte que les première et deuxième extrémités du premier élément flexible allongé se déplacent linéairement, amenant l'un des premier ou deuxième câble de traction à se déplacer dans une première direction et amenant l'autre des premier ou deuxième câble de traction à se déplacer dans une deuxième direction opposée, et
où une partie distale (239) d'un gastroscope (130) couplée aux premier et deuxième câbles de traction est fléchie en fonction des déplacements des premier et deuxième câbles de traction.

2. Sonde d'échocardiographie transœsophagienne selon la revendication 1, comprenant en outre:
une deuxième entrée de commande située à l'extérieur du cadre de confinement;
un deuxième arbre situé à l'intérieur du cadre de confinement et couplé à la deuxième entrée de commande;
un deuxième élément flexible allongé comprenant une première extrémité et une deuxième extrémité, où le deuxième élément flexible allongé est enroulé autour du deuxième arbre;
une troisième fixation de câble de traction couplée à la première extrémité du deuxième élément flexible allongé;
une quatrième fixation de câble de traction couplée à la deuxième extrémité du deuxième élément flexible allongé;
un troisième câble de traction fixé à la troisième fixation de câble de traction; et
un quatrième câble de traction fixé à la quatrième fixation de câble de traction,
où, lorsque la deuxième entrée de commande tourne, le deuxième arbre tourne de telle sorte que les première et deuxième extrémités du deuxième élément flexible allongé se déplacent linéairement, amenant l'un des troisième ou quatrième câbles de traction à se déplacer dans la première direction, et amenant l'autre des troisième ou quatrième câbles de traction à se déplacer dans la deuxième direction opposée, et où la partie distale du gastroscope couplée aux troisième et quatrième câbles de traction est fléchie en fonction des déplacements des troisième et quatrième câbles de traction.

3. Sonde d'échocardiographie transœsophagienne selon la revendication 2, comprenant en outre un commutateur de frein monolithique qui, lorsqu'il est tourné dans une première position, applique une résistance à la rotation des premier et deuxième arbres et, lorsqu'il est tourné dans une deuxième position, permet aux premier et deuxième arbres de tourner sans résistance.

4. Sonde d'échocardiographie transœsophagienne selon la revendication 3, dans laquelle au moins l'un parmi les entrées de commande, les arbres, les éléments flexibles allongés, le commutateur de frein ou le cadre de confinement est non conducteur.

5. Sonde d'échocardiographie transœsophagienne selon la revendication 4, dans laquelle au moins l'un parmi les entrées de commande, les arbres, le commutateur de frein ou le cadre de confinement est en plastique moulé.

6. Sonde d'échocardiographie transœsophagienne selon la revendication 2, dans laquelle les premier et deuxième éléments flexibles allongés sont constitués de composés élastomères moulés sur des éléments de renfort en nylon.

7. Sonde d'échocardiographie transœsophagienne selon la revendication 2, dans laquelle la première entrée de commande dirige la flexion antérieure et postérieure de la partie distale du gastroscope, et la deuxième entrée de commande dirige la flexion gauche/droite du gastroscope.

8. Sonde d'échocardiographie transœsophagienne selon la revendication 2, dans laquelle les premier et deuxième éléments flexibles allongés sont des courroies de friction.

9. Sonde d'échocardiographie transœsophagienne selon la revendication 2, dans laquelle les premier et deuxième arbres comprennent des engrenages ou pignons, et dans laquelle les premier et deuxième éléments flexibles allongés sont des courroies en échelle de synchronisation s'interfaçant avec les engrenages ou pignons,
ou
dans laquelle les premier et deuxième arbres comprennent des engrenages ou pignons, et dans laquelle les premier et deuxième éléments flexibles allongés sont des courroies de synchronisation s'interfaçant avec les engrenages ou pignons,
ou
dans laquelle les premier et deuxième arbres comprennent des engrenages ou pignons, et dans laquelle les premier et deuxième éléments flexibles allongés sont des bandes d'entraînement s'interfaçant avec les engrenages ou pignons.

10. Sonde d'échocardiographie transœsophagienne selon la revendication 2, dans laquelle le premier arbre et le deuxième arbre comprennent chacune une douille, et dans laquelle le premier élément flexible allongé est un câble de poulie ancré au premier arbre par une fixation à bille sertie ou à vis s'insérant dans la douille dans le premier arbre, et dans laquelle le deuxième élément flexible allongé est un câble de poulie ancré au deuxième arbre par une fixation à bille sertie ou à vis s'insérant dans la douille du deuxième arbre.

11. Sonde d'échocardiographie transœsophagienne selon la revendication 2, dans laquelle les fixations du câble de traction sont formées directement dans les éléments flexibles allongés.

12. Sonde d'échocardiographie transœsophagienne selon la revendication 2, dans laquelle les fixations du câble de traction comprennent chacune un mécanisme de tension fileté.

13. Sonde d'échocardiographie transœsophagienne selon la revendication 2, comprenant en outre un boîtier de poignée externe.

14. Sonde d'échocardiographie transœsophagienne selon la revendication 2, dans laquelle les première et deuxième arbres sont coaxiales.

15. Sonde d'échocardiographie transœsophagienne selon la revendication 2, comprenant en outre un arbre intérieur flottant non rotatif fixé au cadre de confinement et situé entre les premier et deuxième arbre pour limiter le couplage transversal de mouvement entre le premier et le deuxième arbre.
